(19) Europäisches Patentamt
European Patent Office
Office européen des brevets

(11) Publication number: **0 107 277**
**B1**

(12) **EUROPEAN PATENT SPECIFICATION**

(45) Date of publication of patent specification: **28.05.86**

(51) Int. Cl.$^4$: **A 01 N 59/12,** A 61 L 15/03 // C08F218/08, C08F226/10

(21) Application number: **83304481.1**

(22) Date of filing: **03.08.83**

(54) **Pliable films of polymeric halogen complexes.**

(30) Priority: **30.09.82 US 430572**

(43) Date of publication of application:
**02.05.84 Bulletin 84/18**

(45) Publication of the grant of the patent:
**28.05.86 Bulletin 86/22**

(84) Designated Contracting States:
**DE FR GB IT**

(56) References cited:
**FR-A-1 252 512**
**FR-A-1 252 612**
**FR-A-2 295 757**
**US-A-3 608 070**

**CHEMICAL ABSTRACTS, vol. 77, no. 26, 25th December 1972, no. 165869t, Columbus, Ohio, USA V.M. POZDNYAKOV et al.: "Production of fibers for medical use based on vinyl alcohol-vinylpyrrolidione copolymer and mixtures of their homopolymers"**

(73) Proprietor: **GAF CORPORATION**
**140 West 51st Street**
**New York New York 10020 (US)**

(72) Inventor: **Barabas, Eugene S.**
**41 Stanie Brae Drive**
**Watchung New Jersey 07060 (US)**
Inventor: **Waxman, Burton H.**
**3 Wenonah Avenue**
**Rockaway New Jersey 07866 (US)**

(74) Representative: **Ford, Michael Frederick et al**
**MEWBURN ELLIS & CO. 2/3 Cursitor Street**
**London EC4A 1BQ (GB)**

**EP 0 107 277 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is directed to certain novel polymeric-halogen complexes and methods for producing the same. More particularly, the present invention is directed to pliable halogen complexes of vinyl acetate/vinylpyrrolidone copolymer useful as an antimicrobial medium.

### Background of the Invention

For many years, the halide complexes of polyvinylpyrrolidone have been employed to treat wounds or infectious diseases or to otherwise provide an antimicrobial environment. These materials however are not entirely satisfactory since they tend to cake or, when applied as a film are subject to cracking which causes irritation and reduces efficiency. Alternatively, the teaching of U.S. Patent No. 3,907,720 describes insoluble iodine complexes of homo and copolymers of vinylpyrrolidone which due to their cross-linked character are incapable of forming films.

FR-A-1252612 describes iodine complexes of copolymers of N-vinylpyrrolidone and vinyl monomer which contain a major proportion (60 wt % or more) of N-vinylpyrrolidone. Vinyl acetate is mentioned as a comonomer. These complexes are not pliable. It is not suggested that they should be used in treating wounds, or infectious diseases.

It is an object of this invention to provide a pliable antimicrobial film which can be applied to a bandage or which, itself, can be the binding or bandaging agent over a wound.

Another object of this invention is to provide an adhesive antimicrobial film of a novel polymeric iodine or bromine complex which can be used to disinfect surfaces, medical equipment, wounds or fungicidal infections.

Still another object of this invention is to provide an economical and commercially desirable film in a stable pliable form having good bactericidal properties.

### The Invention

According to this invention there is provided an antimicrobial film containing a copolymer 50 to 80 wt % vinyl acetate and 20 to 50 wt % vinylpyrrolidone, which copolymer is combined with iodine and/or bromine to provide at least 2 weight %, preferably 2% to 25 weight %, available halide in the final product. The polymer contains random distribution of

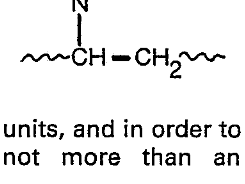 and 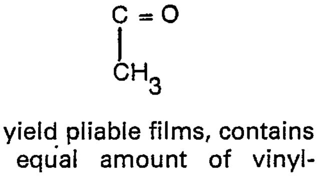

units, and in order to yield pliable films, contains not more than an equal amount of vinylpyrrolidone, preferably a minor amount, e.g. not more than 40 weight % with respect to vinyl acetate. The preferred complexed polymer contains between 5 and 20 wt % available halide as iodine, bromine or a mixture of iodine and bromine. The weight percent of monomers in the copolymer, i.e. at least 50 wt % vinyl acetate is critical in achieving a complexed film having good pliability, and adhesion.

The preparation of the present complex is relatively simple and preferably involves the preformation of the copolymer. In general the copolymer formed can be made by solution, suspension, precipitation or emulsion polymerization by mixing the monomers in the above recited proportions in the presence of a free radical catalyst generally employed in vinyl polymerizations such as peroxides, peresters or percarbonates, etc. or azo catalysts, e.g. azobisisobutyronitrile, azo-bis-dimethylvaleronitrile, hydrogen peroxide, t-butylperoxide, t-butyl peroxy pivalate and diisopropyl percarbonate. Alternatively the polymer can be made by UV or gamma radiation. The polymerization reaction is effected at a temperature between about 0°C. and 100°C., preferably between about 10°C. and 80°C. under a pressure of from about 101.3 to 506.5 kPa (about 1 to about 5 atmospheres) for a period of from 0.25 to 10 hours, preferably from about 2 to about 8 hours with mild agitation. The product when prepared in alcoholic solution can be used as is to prepare the halogen complex. The copolymer solution can then be mixed with an inert diluent, to a concentration of between about 20 wt. % and about 70 wt. %. Suitable diluents include water, ethanol, isopropanol, methanol, depending on ultimate use of the complex. The diluted copolymer is then charged to a reactor maintained at a temperature of between about 20°C. and about 100°C., preferably between about 70°C. and 90°C. under atmospheric pressure, wherein it is contacted with a 10% to 50% solution of the halogen, e.g. halogen in an alcohol solution. The resulting mixture is agitated at the reaction temperature for a period of from 1 to 14 hours during which complex is formed. The complex product, after dilution with water and subsequent azeotropic distillation, is recovered as a viscous, liquid having a Brookfield visocsity of between about 100 and about 300 mPas (cps). Alternatively, nascent iodine and/or bromine can be dry mixed with the preformed polymer in the required amount to produce the present pliable films of this invention.

The product can be used without further purification in its viscous state for application as a coating or it can be diluted with an inert solvent to a concentration of between about 2% and about 50%, preferably between about 5% and about 20% for use as a liquid or aerosol spray to be applied to the desired substrate such as human skin, a fabric such as gauze employed in bandages, glass or metal surfaces, to form a pliable, stable, non-porous disinfectant coating.

The complexes of the present invention can

also be used for agricultural purposes such as seed coating with thin films to prevent fungus infection.

Suitable solvents for dilution of the halogen complex include water, ethanol, methanol, isopropanol or any other inert solvent commensurate with the use of the product.

The complexed polymers of this invention not only possess the desirable ability to form pliable films, but also provide antiseptic, antimicrobial agents which reduce toxicity, irritation and skin sensitivity effects of iodine or bromine *per se*. The complexed products have lower halogen vapor pressure than iodine itself and enhanced stability and exhibit a longer antimicrobial effect than iodine, bromine or the powdered or granular PVP/halogen complexes.

Having thus described the invention, reference is now had to the following examples which set forth preferred embodiments but which are not to be construed as limiting the scope of the invention.

Example 1

Into a 2 liter kettle equipped with a mechanical stirrer, a reflux condenser, thermometer, dropping funnel and gas inlet tube, which had been purged for 15 minutes with nitrogen, was introduced 500 grams Ethanol 3A denatured with methanol in 20:1 by volume ethanol: methanol ratio. Vinyl acetate and vinyl pyrrolidone (250 grams of each) were mixed in a separate flask and the monomeric mixture was placed in the dropping funnel. Ten percent of the monomer mixture (50 grams) was added in one batch to the alcohol and agitation was started followed by the addition of 0.25 grams of initiator (azo-bis-isobutyronitrile). The temperature of the reaction mixture was raised to gentle boiling (about 75°C.), at which temperature the mixture was stirred for 15 minutes. The addition of monomer was begun and completed within 2 hours during reflux and reflux conditions were maintained for an additional hour. At this time 0.05 grams of azo-bis-isobutyronitrile was added and the mixture held at reflux for 1 hour longer. The polymer was then cooled to room temperature and discharged. Analysis was as follows: Total Solids = 50.28%; Residual VP = 0.20%; Residual VA = 0.32% and Relative Viscosity (1%) = 1.274.

Example 2

The vinylpyrrolidone/vinyl acetate copolymer of Example 1 (200 g. 50% active in ethanol) was charged to a 1 liter kettle equipped with mechanical stirrer, reflux condenser, gas inlet tube and thermometer. The kettle was heated to 85°C. and an iodine solution (20% in ethanol) was aded in a 2 minute period with stirring. After stirring for 90 minutes at 85°C. the reaction was completed and the reflux condenser was replaced by a Liebig condenser. Distillation was started with simultaneous gradual addition of 480 ml distilled water. The temperature increased to 96°C. after about 350 ml distillate was removed. The distilland was cooled to 85°C. and maintained at this temperature for 60 minutes, after which the system was cooled to room temperature and the product was discharged and recovered as a low viscosity solution. The product analyzed as follows:

| | | |
|---|---|---|
| Total Solids | : | 30.20% |
| Brookfield V:- | : | 160 mPas (cps) (3/30) |
| pH | : | 1.7 |
| Available Iodine in the solution | : | 2.07% |
| Stability | : | 4.51% 5.10% |

The film cast from the complexed copolymer solution was compared with products similarly prepared, 60/40 and 20/80 VP/VA complexed copolymer. The polymer film prepared from the complex of Example 2 was significantly more pliable than that prepared from the complexed 60/40 polymer but less pliable than that prepared from the complexed 20/80 polymer. The following Table provides a further comparison of VP/VA iodine complex flexibility, based on a scale of 0 to 5.

TABLE

| % VA | % VP | Film Former | Flexibility |
|---|---|---|---|
| 0 | 100 | No | 0 |
| 30 | 70 | Yes | 1 |
| 50 | 50 | Yes | 3 |
| 70 | 30 | Yes | 5 |

Example 3

About 5 g. of the complexed polymer solution of Example 2 was placed on a clean dry glass plate and a film was cast with the aid of a 1.27 mm (50 mil) doctor-blade. The water-soluble film was allowed to air-dry overnight. It was then tested and found to coat the glass well, without any tendency to deteriorate. The peeled-off film was found to be pliable and non-porous. It was observed for several days, without any physical alteration.

Example 4

The procedure of Example 2 was repeated except that a bromine solution (20% in ethanol) replaced the iodine solution. The resulting complexed product, when applied to a glass substrate exhibited the same pliability of a homogeneous continuous film which adhered to the glass substrate.

Example 5

When the complexed products containing 30% VP and 70% VA, prepared by the procedures set forth in Examples 2 and 4 (5 g. of complexed polymer in water) are applied as a 0.127 mm (5 mil) film to a gauze bandage pad 76.2 × 76.2 mm (3 × 3 inches) and dried, the pad remains pliable without rupture of the complexed polymer film.

Example 6

The complexed products containing 30% VP and 70% VA, prepared by the process set forth in Examples 2 and 4 also can be formulated as an aerosol spray and sprayed over a cut or wound of any living organism to provide a pliable antiseptic barrier. In this manner the complexed polymer sprays can be applied to the skin of a human or animal or to plant tissue. The sprays can also be applied in solution or in a spray to seeds as protection against mildew, and other deleterious infections, or can be employed to coat inner surfaces of bags or containers used to transport seeds, fruit, vegetables, and other crops.

The aerosol spray is prepared by dissolving 5 g. of complexed polymer in 50 g. water, then adding 45 g. dimethylether.

It is to be understood that mixtures of iodine and bromine complexes of the present copolymer can be substituted in Examples 3—6 to provide substantially the same results.

**Claims**

1. A film forming complexed antimicrobial polymer comprising a copolymer of from 50 to 80 weight % of vinyl acetate and from 20 to 50 weight % vinyl pyrrolidone, said copolymer being complexed with iodine or bromine, to provide a product having at least 2 weight % available iodine or bromine.

2. A pliable film forming complexed, antimicrobial polymer consisting essentially of a copolymer of from 50 to 80 weight % of vinyl acetate and from 20 to 50 weight % of vinyl pyrrolidone, said copolymer being complexed with iodine or bromine to provide a product having between from 2 to 25 weight % available iodine or bromine.

3. The complexed polymer of claim 2 wherein the copolymer contains from 60 to 80 weight % of vinyl acetate and from 20 to 40 weight % of vinyl pyrrolidone complexed with iodine to provide a product having between 5 and 25 weight % available iodine.

4. The process for the preparation of the complexed polymer of claim 1 which comprises copolymerizing from 50 to 80 weight % of vinyl acetate with from 20 to 50 weight % vinyl pyrrolidone in the absence of a cross-linking agent, at a temperature of from 0° to 100°C under between 101.3 and 506.5 kPa (1 and 5 atmospheres) pressure and then mixing the non-cross linked copolymer with iodine or bromine for a period of from 1 to 14 hours at a temperature of between 20°C and 100°C.

5. The process of claim 4 wherein the copolymerization reaction is carried out in the presence of an inert liquid medium.

6. The process of claim 5 wherein the inert liquid medium is a low molecular weight alcohol and/or water.

7. A product of manufacture comprising a wound dressing coated with the complexed film forming, antimicrobial copolymer of any one of claims 1 to 3.

8. A wound dressing comprising an aerosol spray containing an effective antimicrobial amount of the complexed, antimicrobial copolymer of any one of claims 1 to 3.

9. The process of disinfecting a surface which comprises applying thereto an effective amount of the film forming, complexed, antimicrobial polymer of any one of claims 1 to 3.

10. The process of protecting seeds or fruit from fungicidal damage by applying to the seed, fruit or citus thereof an effective amount of the film forming, complexed, antimicrobial polymer of any one of claims 1 to 3.

**Patentansprüche**

1. Filmbildendes, komplexiertes, antimikrobielles Polymer aus einem Copolymer mit 50 bis 80 Gew.-% Vinylacetat und 20 bis 50 Gew.-% Vinylpyrrolidon, wobei das Copolymer mit Jod oder Brom komplexiert ist, um ein Produkt zu ergeben, das mindestens 2 Gew.-% verfügbares Jod oder Brom aufweist.

2. Einen biegsamen Film bildendes, komplexiertes, antimikrobielles Polymer, das im wesentlichen aus einem Copolymer aus 50 bis 80 Gew.-% Vinylacetat und 20 bis 50 Gew.-% Vinylpyrrolidon besteht, wobei das Copolymer mit Jod oder Brom komplexiert ist, um ein Produkt zu ergeben, das zwischen 2 und 25 Gew.-% verfügbares Jod oder Brom aufweist.

3. Das komplexierte Polymer nach Anspruch 2, bei dem das Copolymer 60 bis 80 Gew.-% Vinylacetat und 20 bis 40 Gew.-% Vinylpyrrolidon enthält und komplexiert ist mit Jod, um ein produkt zu ergeben, das zwischen 5 und 25 Gew.-% verfügbares Jod aufweist.

4. Verfahren zum Herstellung des komplexierten Polymers nach Anspruch 1, das das Copolymerisieren von 50 bis 80 Gew.-% Vinylacetat mit 20 bis 50 Gew.-% Vinylpyrrolidon bei Abwesenheit eines Vernetzungsmittels bei einer Temperatur von 0 bis 100°C und unter einem Druck von 101,3 bis 506,5 kPa (1 bis 5 Atmosphären) und das nachfolgende Vermischen des nicht vernetzten Copolymers mit Jod oder Brom für eine Dauer von 1 bis 14 Stunden bei einer Temperatur von 20 bis 100°C umfaßt.

5. Verfahren nach Anspruch 4, bei dem die Copolymerisation in Gegenwart eines inerten flüssigen Mediums ausgeführt wird.

6. Verfahren nach Anspruch 5, bei dem das inerte flüssige Medium ein Alkohol geringen Molekulargewichtes und/oder Wasser ist.

7. Herstellungsprodukt, umfassend einen

Wundverband, der mit dem komplexierten, filmbildenden, antimikrobiellen Copolymer nach irgendeinem der Ansprüche 1 bis 3 überzogen ist.

8. Wundverband umfassend ein Aerosol-Spray, das eine wirksame antimikrobielle Menge des komplexierten, antimikrobiellen Copolymers nach irgendeinem der Ansprüche 1 bis 3 enthält.

9. Verfahren zum Desinfizieren einer Oberfläche durch Auftragen einer wirksamen Menge des filmbildenden, komplexierten, antimikrobiellen Polymers nach irgendeinem der Ansprüche 1 bis 3.

10. Verfahren zum Schützen von Samen oder Früchten vor Pilzbefall durch Aufbringen einer wirksamen Menge des filmbildenden, komplexierten, antimikrobiellen Polymers nach irgendeinem der Ansprüche 1 bis 3 auf den Samen, die Zitrus- oder anderen Früchte.

**Revendications**

1. Polymère antimicrobien complexé pour la formation de films, comprenant un copolymère de 50 à 80% en poids d'acétate de vinyle et de 20 à 50% en poids de vinylpyrrolidone, ledit copolymère étant complexé avec de l'iode ou du brome, pour fournir un produit ayant au moins 2% en poids d'iode ou de brome disponible.

2. Polymère antimicrobien complexé pour la formation de films pliables constitué essentiellement d'un copolymère de 50 à 80% en poids d'acétate de vinyle et de 20 à 50% en poids de vinylpyrrolidone, ledit copolymère étant complexé avec de l'iode ou du brome pour fournir un produit ayant entre 2 et 25% en poids d'iode ou de brome disponible.

3. Polymère complexé selon la revendication 2, dans lequel le copolymère contient de 60 à 80% en poids d'acétate de vinyle et de 20 à 40% en poids de vinylpyrrolidone complexé avec de l'iode pour fournir un produit ayant entre 5 et 25% en poids d'iode disponible.

4. Procédé de préparation du polymère complexé selon la revendication 1, comprenant la copolymérisation de 50 à 80% en poids d'acétate de vinyle avec de 20 à 50% en poids de vinylpyrrolidone en l'absence d'un agent de formation de liaisons croisées, à une température entre 0 et 100°C sous une pression comprise entre environ 100 000 et 500 000 Pa (1 et 5 atmosphères), puis le mélange du copolymère sans liaison croisée avec de l'iode ou du brome pendant une période de temps entre une et 14 heures à une température comprise entre 20°C et 100°C.

5. Procédé selon la revendication 4, dans lequel la réaction de copolymérisation est mise en oeuvre en présence d'un milieu liquide inerte.

6. Procédé selon la revendication 5, dans lequel le milieu liquide inerte est un alcool à faible poids moléculaire et/ou de l'eau.

7. Produit de manufacture comprenant un pansement de blessure revêtu du copolymère antimicrobien complexé formant film selon l'une quelconque des revendications 1 à 3.

8. Pansement de blessure comprenant un produit de pulvérisation en aérosol contenant une quantité antimicrobienne efficace du copolymère antimicrobien complexé selon l'une quelconque des revendications 1 à 3.

9. Procédé de désinfection d'une surface comprenant l'application à celle-ci d'une quantité efficace du polymère antimicrobien complexé formant film selon l'une quelconque des revendications 1 à 3.

10. Procédé de protection de graines ou de fruits contre les champignons par application aux graines, ou aux fruits d'une quantité efficace du polymére antimicrobien complexé formant film selon l'une quelconque des revendications 1 à 3.